# EUROPEAN PATENT SPECIFICATION

(11) **EP 3 781 031 B1**
(45) Date of publication and mention of the grant of the patent: **29.03.2023**
(21) Application number: 20724937.6
(22) Date of filing: 27.04.2020
(51) Int. Cl.: A61B 5/05, A61B 5/06, A61B 6/12, A61B 6/00, A61B 90/00

(54) **DUAL MODE MARKER AND TRACER DETECTION SYSTEM**
DUALMODUS-SYSTEM FÜR MARKER- UND TRACERERKENNUNG
SYSTÈME DE DÉTECTION DE MARQUEUR ET DE TRACEUR BIMODE

(30) Priority: 29.04.2019 GB 201905988
(43) Date of publication of application: 24.02.2021
(73) Proprietor: Endomagnetics Ltd., Cambridge CB4 0WN (GB)
(72) Inventor: HARMER, Quentin, Cambridgeshire CB4 0WS (GB); GONZALEZ-CARVAJAL, John, Cambridgeshire CB4 0WS (GB)
(74) Representative: Abel & Imray LLP
(86) International application number: PCT/IB2020/053929
(87) International publication number: WO 2020/222106

(56) References cited:
- WO-A1-2016/012556
- WO-A1-2016/142675
- WO-A1-2018/045465
- WO-A2-2009/128062
- US-A1- 2005 255 044
- US-A1- 2006 106 306
- US-A1- 2014 018 663

## Description

### Background

Gamma detection systems comprising a handheld probe in communication with a base unit which is used to detect sentinel lymph node tracers in a sentinel lymph node biopsy procedure are known in the art. A radiopharmaceutical tracer is injected near a lesion of interest, travels to the lymph nodes draining the lesion and accumulates there allowing detection. A gamma detection system where the probe communicates via a wireless link to the base station is described in US9671504 (Neoprobe Corp).

Magnetic sentinel node tracer detection systems are also known in the art for example US8174259 which describes a handheld magnetic detection probe in communication with a base unit for sentinel lymph node tracer detection. A magnetic tracer comprising superparamagnetic iron-oxide nanoparticles is injected near the lesion, travels to the lymph nodes and accumulates within the nodes allowing the nodes to be identified using magnetic detection.

Also known are systems for lesion localization in which a 'non-palpable' lesion (one that is too small to feel or see during surgery) for example a cancerous tumour or benign lesion is marked with an implantable marker prior to surgery, and a handheld probe is used during surgery to guide the surgeon to the lesion and allow accurate excision of the lesion while minimizing the amount of healthy tissue that is removed. A number of marker detection technologies are known for localizing lesions via detection of an implantable marker:
- US 2017/252124 (Cianna Medical) discloses a localization system which uses a combination of radio frequency (RF) electromagnetic signals and infra red (IR) radiation to detect a marker in the form of an implantable reflector. The RF signals are also referred to interchangeably as radar signals.
- US 2015/264891 (Health Beacons) discloses a further system based on radio frequency identification (RFID) tags that have been used as identity markers for pets and livestock. The RFID tag is used to mark a lesion and an RFID probe is used to detect the marker.
- US20170095315 (Elucent Medical) discloses a tag detection system where the tag is activated by a magnetic field and the tag generates magnetic frequencies that are detectable by a witness stations that detect the tag and communicate with a base unit.
- A further approach is discussed in the Applicant's earlier published patent applications (for example, WO 2011/067576, WO 2014/013235 and WO 2014/140567) and uses magnetic fields and a magnetic marker with high magnetic susceptibility. A handheld probe generates an alternating field which excites a magnetically responsive marker and detects the responding magnetic field.
- US7881775 discloses a radioactive seed localisation system where a seed containing a radioactive isotope is placed in the tumour and a handheld gamma probe is used to locate the seed and the tumour for excision.
- US 2006/106306 discloses instruments and instrumental techniques for locating cancer cells in lymph nodes which utilize a radiation detector operatively connected to an ultrasound probe for locating the exact position of radiation tagged tissue followed by placement of a biopsy device.
- WO 2016/012556 discloses a system for medical imaging comprises a functional detector, wherein a control unit configured to calculate the position of the respective other of a functional detector and an ultrasonic probe as captured by an optical camera.
- US 2005/255044 discloses a combined modality imaging system includes a first imaging device of a first modality and a second imaging device of a second modality that is different from the first modality. The imaging devices are both adapted to interact with a contrast agent that includes a deformable particle that has a geometry that varies in response to an emission from the first imaging device and a fluorescent component adapted to emit electromagnetic radiation that is detectable by the second imaging device.

While imaging technologies such as ultrasound, MRI or fluorescence imaging can be used to image cancer lesions or lymph nodes, the technologies used to detect lesion localisation markers or to detect sentinel lymph node tracers are non-imaging detection technologies, and the systems described herein are non-imaging detection systems.

Typically, in breast cancer surgery it is common for both sentinel node tracer detection and lesion localisation marker detection techniques to be used together sequentially, where the lesion is located and excised and the sentinel nodes are located and excised as part of the same procedure. The order of use of the two techniques may vary depending on the surgical approach being used.

In some cases, it is possible for the same detection system to detect both sentinel nodes and lesion markers. This requires a liquid tracer for sentinel node biopsy and a solid marker for lesion localization, both of which can be detected using the same technology. For example, a liquid radiopharmaceutical tracer and implantable radioactive seeds can both be detected by a gamma probe. Also, a magnetic nanoparticle tracer and a magnetic seed can be detected by a magnetic probe.

However, to date, each of these systems contains only one non-imaging detection technology, and this can result in a sub-optimal result for the user. For example, radiopharmaceutical tracers are the standard of care in many hospitals for sentinel lymph node detection, but radioactive seeds are much less widely used because of the radiological regulations about their use which require an onerous chain of custody and safety precautions to be implemented before the radioactive seeds can be used. As a result, hospitals may have a gamma probe detection system for sentinel lymph node detection procedures, but an alternative system for lesion localization such as a magnetic or radar-based system. This means that there are two base units in the operating room taking up valuable space. Surgeons also need to use both systems in a typical breast cancer procedure where both lesion localization and sentinel node biopsy are performed in the same procedure. This requires them to learn how to use both systems in the first instance, but then to use two potentially very different user interfaces to receive the detection information from two probes all within the same procedure, even though the probes are at an essential level both performing a similar function of locating a marker or tracer in the body.

Therefore, there is a need for a non-imaging detection system that integrates both the function of marker detection for lesion localisation and the function of tracer detection for sentinel lymph node mapping into a single system. The present invention aims to address this need.

### Summary of the Invention.

According to the present invention there is provided a non-imaging detection system for locating markers or tracers in the body comprising, the system comprising:
a base unit and at least two detection probes, wherein a first detection probe uses a first non-imaging detection technology to detect an implantable marker, preferably for lesion localisation and a second detection probe uses a second different, non-imaging detection technology to detect an injectable tracer, preferably for lymph node detection;
and wherein the base unit is capable of receiving detection signals and providing detection information to a user from both probes.

Lesion localisation comprises detection technology to detect and locate markers marking the site of a lesion of interest, such as a cancerous tumour. Lesion markers are generally solid markers implanted via a needle to mark a region of interest in tissue. Lymph node detection comprises detection technology to detect and locate a tracer that has accumulated in lymph nodes of interest, such as those draining from a cancerous tumour. Tracers used for lymph node detection are generally liquids, or colloidal suspensions of nanosized particles. Preferably the particles have a hydrodynamic diameter of between 5 and 100 nanometres, and more preferably between 5 and 60 nanometres.

Detection technology is defined as a non-imaging means of detecting or energizing and detecting a marker or tracer. A detection technology is typically based on one or more physical effects such as gamma rays and gamma ray detection or magnetic detection. Various non-imaging detection technologies can be envisaged within the scope of the invention including detection technologies that use any of these effects:
- Detection of radiation or gamma rays;
- Generation and detection of alternating magnetic fields;
- Generation and detection of DC magnetic fields;
- Detection of DC magnetic fields;
- Generation and detection of induced Eddy currents;
- Excitation and or detection using radio-frequency waves or radar waves or other electromagnetic waves or signals;
- Radio-frequency identification (RFID) tag detection; and
- Infra-red radiation excitation and detection.

A detection technology may use a single physical effect. For example gamma detection or may use a combination of effects such as radar and infra-red energy as described in US 2017/252124.

One or more detection probes can be envisaged each able to detect via one of the detection modalities. Most usually it would be envisaged that two or more probes are supplied, each using one detection modality but all communicating with the same base unit.

In theory more than one detection technology could be integrated into the same handheld probe. However, in practice this is challenging due to the space constraints within a probe and the conflicting requirements of different detection technologies, and so it is usually more convenient to have a separate probe for each technology.

For example, a gamma detector requires a crystal radiation sensor to be positioned at or near to the distal tip of the handheld probe, while an RF detector would require an antenna or waveguide also in the distal tip region of the probe. Thus, if arranged in the same probe, they would a) have the potential to interfere with one another, and b) one or both would need to be positioned sub-optimally in order to make space for the other.

The base unit preferably has components common to both detection technologies and components specific to each different detection technology. For example, components common to both detection technologies may be selected from the group consisting of: a main processing unit; a signal processing unit; a display; an audio output; an analogue to digital convertor (ADC); a digital to analogue convertor (DAC); data storage memory; user controls and caseworks or any combinations thereof.

Dependent on the type of marker or tracer, the base unit may be provided with a drive signal source for providing a drive signal to one or both probes to excite an energy source in the probe for exciting the marker or tracer.

The marker or tracer emits a signal which is detected by a sensor in the first or second probe which is transmitted to and received by the base unit.

Examples of non-imaging marker detection technology used in the marker detection probe are selected from: gamma detection; magnetic detection; electromagnetic detection; RFID detection; DC magnetic field sensing; AC magnetic susceptometry; DC magnetic excitation and detection; resonant magnetic tag detection; Radar detection; and infra-red detection, or any combinations thereof.

Examples of non-imaging tracer detection technology used in the tracer detection probe are selected from: gamma detection; magnetic detection; DC magnetic field sensing; and AC magnetic susceptometry.

Preferably, the system is provided with a mode select switch to switch between a marker detection probe mode and a tracer detection probe mode. The switch may comprise a switch or dial on the base unit, a footswitch, a control on the probe or be via voice or gesture control.

Preferably, the base unit includes a visual or audio display providing a different theme dependent upon the mode selected to aid differentiation between the modes of detection.

The base unit may include an amplifier to amplify the detection signals received from the detection probes and other components to improve signal processing, such as appropriate filters.

Preferably, the base unit receives signals from one or both probes to provide an output indicating at least one of the position of the marker or tracer and the distance of the marker or tracer from the probe, or the proximity of the marker or tracer to the probe.

In one embodiment the probe identifies when it is about to be in-use, via a motion, touch, gesture or other sensor on the probe, and communicates with the base unit an identification signal so that the base unit selects the relevant detection mode for that detection probe.

In one embodiment, one or more of the handheld probes is connected to the base unit via a wire or cable and one or more other handheld probes communicates with the base unit via a wireless connection.

In a preferred embodiment, the system comprises a base unit, a handheld magnetic detection probe connected via a cable to the base unit, and a handheld cordless gamma probe that communicates with the base unit via a wireless connection.

In another preferred embodiment, the system comprises a base unit, a handheld radar-based detection probe connected via a cable to the base unit, and a handheld cordless gamma probe that communicates with the base unit via a wireless connection.

In another preferred embodiment, the system comprises a base unit, a handheld RFID detection probe connected via a cable to the base unit, and a handheld cordless gamma probe that communicates with the base unit via a wireless connection.

In a further embodiment, controls are provided on the probe to allow the user to activate the probe, reset the display, change the display mode, mute the audio signal or another system function. The controls could be buttons, or other types of switch of gesture sensor.

In a further embodiment, the handheld probe provides haptic feedback to the user to guide the detection of a marker in the body. The haptic feedback may be in the form of clicks, vibration, movement or force.

In a further embodiment, the system further comprises a footswitch for controlling the detection, for example to activate the probe, reset the display, change the display mode, mute the audio signal or another system function. The footswitch may be connected to the base unit via a cable or wire, or may communicate wirelessly with the base unit.

In a further embodiment, the system further comprises a scanning probe or wand able to detect the presence of a marker over a wider area of the body. This scanning probe is used to rapidly scan the patient's body prior to making an incision to locate the regions in which a marker is present prior to more localised, precise detection with a handheld detection probe.

### Brief Description of the Drawings.

For a better understanding of the present invention and to show more clearly how it may be carried into effect, reference will now be made by way of example only, to the accompanying drawings, in which:
Figure 1A shows a schematic diagram of a generalised detection system according to the invention comprising a base unit, a marker detection probe and a tracer detection probe;
Figure 1B is a diagram of a detection system according to an embodiment of the invention, the detection system comprising a base unit, a wireless gamma probe and a wired magnetic probe;
Figure 2 illustrates a further embodiment of a detection system of the invention for locating an implanted marker, the detection system comprising a base unit, an RFID detection probe, a radar-based detection probe and a wireless gamma probe;
Figure 3 illustrates another embodiment of a detection system of the invention comprising a wireless magnetic detection probe of one diameter, a further wireless magnetic detection probe with a smaller diameter and a wireless gamma probe;
Figure 4 illustrates a further embodiment of part of a detection system of the invention comprising a scanning probe or wand connected wirelessly to the base unit.
Figure 5 illustrates a block diagram of one embodiment of a detection system of the invention comprising a magnetic tracer detection probe and a marker detection system using a different detection technology;
Figure 5A illustrates a block diagram of a magnetic tracer detection probe for use with the invention using one or more drive and sense coils;
Figure 6 illustrates a block diagram of an embodiment of the detection system of the invention comprising a radioactive tracer detection probe and a marker detection system using a different detection technology;
Figure 6A illustrates a block diagram of a tracer detection system for use with the invention comprising a radioactive tracer detection technology with a wireless probe;
Figure 6B illustrates a block diagram of a tracer detection system for use with the invention comprising a radioactive tracer detection technology with a wired probe;
Figure 7 illustrates a block diagram of a marker detection system for use with the invention comprising a magnetic detection technology;
Figure 8 illustrates a block diagram of a marker detection system for use with the invention comprising a resonant magnetic tag detection technology;
Figure 9 illustrates a block diagram of a marker detection system for use with the invention comprising an RFID detection technology;
Figure 10 illustrates a block diagram of a marker detection system for use with the invention comprising a radar detection technology;
Figure 11 illustrates a block diagram of a marker detection system for use with the invention comprising magnetic susceptometry detection technology; and
Figure 12 illustrates a block diagram of a marker detection system for use with the invention comprising an Eddy current detection technology.

### Definitions:

The term "microcontroller" refers to a computer central processing unit device that is able to read a program from a computer memory (e.g., ROM or other computer memory) and perform a set of steps according to the program.

The term "computer memory" or shortened to "memory" refers to any storage media readable by a computer processor. Examples of computer memory include, but are not limited to, RAM, ROM and removable storage such as CD ROM, magnetic tapes and hard drives.

The term "transmit" refers to the movement of data in analogue or digital form from one location to another or from one device to another using any suitable means.

The term "ferromagnetic" refers to certain materials that can form permanent magnets or are attracted to magnets. Ferromagnetic materials include one or more of the ferromagnetic elements the most common of which are iron, cobalt and nickel.

### Detailed Description of the Invention.

The present invention relates to a detection system for locating lesions and/or locating sentinel lymph nodes in the body, specifically, a system for detecting implantable markers used to mark a site of interest in the body, and for detecting injectable tracers used for example to mark lymph nodes in the body.

The invention combines two or more non-imaging detection technologies, one technology for marker detection, and one technology for tracer detection. Each technology is associated with a handheld detection probe.

While most of the technologies are suited to detect either markers or tracers, some technologies including radioactive detection and magnetic susceptometry detection, can be used for detecting both markers and tracers. Thus a system may comprise two technologies each of which may detect one of markers or tracers, or both markers and tracers.

One or more detection probes can be envisaged each able to detect via one of the detection modalities. Most usually it would be envisaged that two or more probes are supplied, each using one detection modality but all communicating with the same base unit.

Integrating two or more detection technologies into a system with two or more detection probes communicating with a single base unit provides significant benefits for the users of the system:
- A single unit takes up less space in the operating room than two units and has fewer cables and connections to be made.
- The user only needs to learn a single interface as the controls and some aspects of the display and audio interface can be common to the two detection technologies.
- The user will have the optimal probe for each type of procedure rather than compromising through the use of a single probe with one type of detection technology
- A system with multiple probes provides more flexibility in the choice of technology so that the procedure can be carried out optimally even if one technology is contra-indicated for a particular patient.

The invention will now be described with reference to the accompanying figures illustrating embodiments of the detection system.

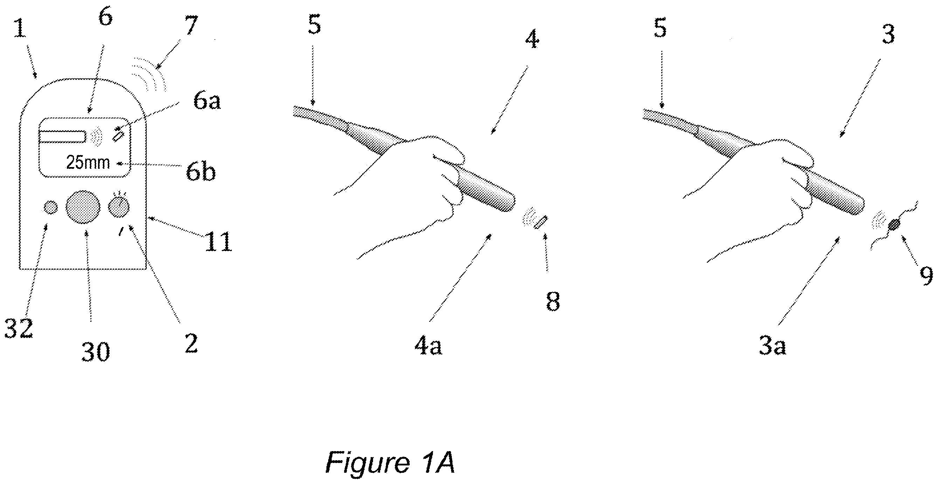

Figure 1A shows a schematic diagram of a basic detection system according to the invention comprising a base unit 1, a tracer detection probe 3 and a marker detection probe 4.

The handheld tracer detection probe 3 employs a tracer detection technology to detect injectable tracers 9 typically used to map or mark lymph nodes. The tracer detection technology is a non-imaging detection technology able to detect the presence of a tracer. Preferably, it is either magnetic detection or radioisotope detection. A second handheld probe, a marker detection probe 4 employs a marker detection technology to detect an implantable marker 8. The marker detection technology is different to the tracer detection technology. The marker detection technology may be any of: DC magnetic field sensing; AC magnetic susceptometry; DC magnetic excitation and detection; resonant magnetic tag; RFID tag sensing; Radar sensing; RF electromagnetic wave sensing; and infra-red sensing. The marker detection technology may be a combination of these technologies for example, radar and infra-red sensing.

Each of the handheld probes 3, 4 communicates with the base unit 1 to transmit the detection signal to the user. The connection to the base unit may be via a wired connection such as a cable 5, or a wireless connection. The base unit may also communicate a drive signal to one or more of the probes via a wired or wireless connection. Where a drive signal is used, the drive signal excites an energy source in the probe which emits an energy field from the distal end 4a, 3a of the probe which excites the marker 8 or tracer 9. The marker or tracer emits a signal which is detected by the sensing technology in the probe and transmitted to the base unit. The drive signal may also be transmitted via an energy field generator that is not in the probe. For example the energy field generator may be in a pad underneath the patient. A mode select switch 2 can be used to switch between detection using a marker detection probe 4 and detection using a tracer detection probe 3.

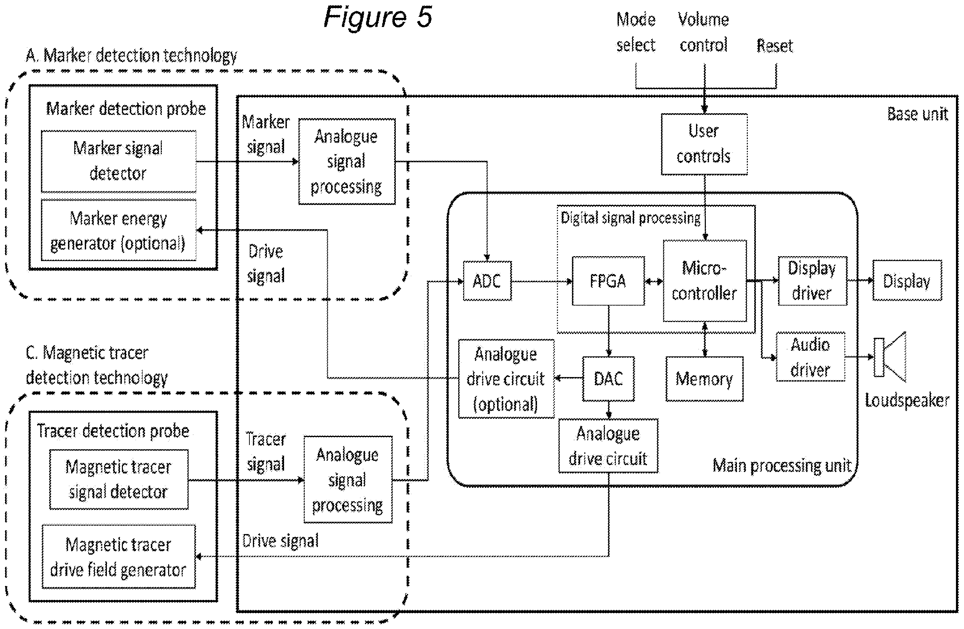

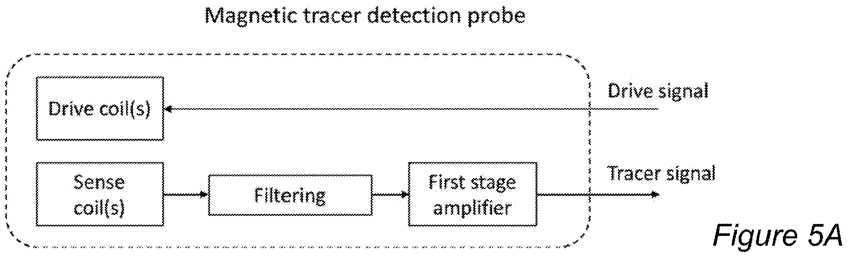

The base unit 1 contains some elements that are specific to one detection technology, for example, parts of the signal processing; and some elements that are common to the two or more detection technologies. Common elements may include any or all of the following: the caseworks or housing 11 of the base unit, the user controls, specifically the volume control 30, mode select switch 2 and where required, the reset switch 32; a display 6 including elements of the graphical display 6a and distance measurement 6b; elements of the audio interface 7; and as shown in Fig 5 but shown in Figure 1A, a display driver, and an audio driver, a display, an audio generator such as a loudspeaker; the main processing unit comprising a microcontroller, a memory unit, a Field Programmable Gate Array (FPGA), an analogue to digital converter (ADC), and a digital to analogue converter (DAC).

Having elements of the base unit that are common to two or more detection technologies reduces the amount of circuitry, electronic components and software in the system and reduces the complexity and cost of the overall system, as well as making the user interface comprising controls, display and audio simpler and easier for users compared with two separate and different systems in separate base units.

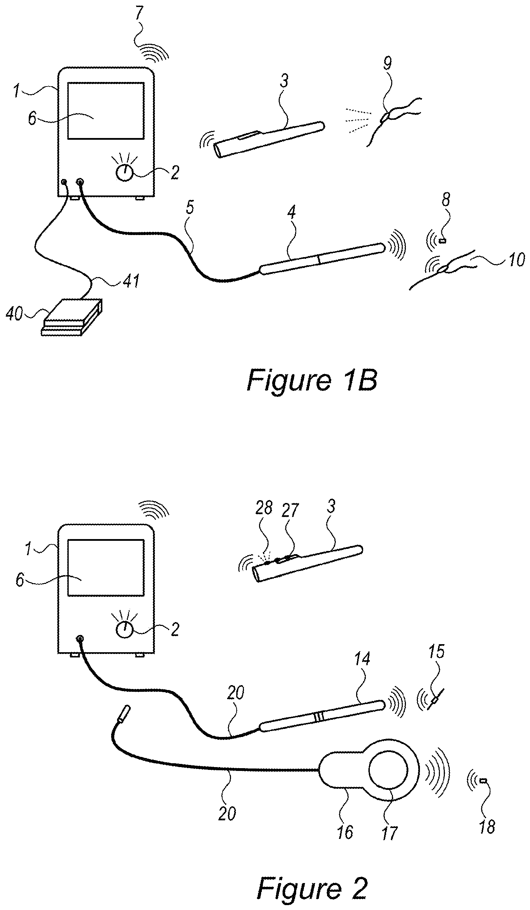

Figure 1B shows an embodiment of a detection system according to the invention that can be used for detecting markers and tracers used in the body to mark lesions and lymph nodes.

The detection system comprises a magnetic detection probe 4 connected to a base unit 1 via a cable or wired connection 5, and a radioactive (gamma) detection probe 3 that can communicate wirelessly with the base unit 1. The base unit 1 further comprises a visual display 6 for providing feedback and information to the user, and a mode switch 2 for switching between detection modes. The base unit contains a receiving module to receive a signal from a probe , and where necessary amplify it, a microprocessor-driven signal processing module and a module to communicate the signal to the user via a visual display 6 or via audio signals 7. For some technologies such as magnetic detection or radar detection, the base unit further comprises means to generate a drive signal to excite the marker via the probe. For example a magnetic detection system may comprise a voltage or current signal generator to generate a waveform to drive a coil in the probe to excite a marker. A radar-based system may contain an RF signal generator to drive an antenna in the probe to excite a marker.

The mode switch 2 can be used to switch between detection using the magnetic probe 4 and detection using the gamma probe 3.

The detection system may further comprise a footswitch 40 connected to the base unit 1 via a cable 41 or wirelessly. The footswitch may control one or more functions of the system, for example to activate the probe, reset the display, change the display mode, mute the audio signal or another system function.

The system may be used to locate a magnetic marker 8 in the body by use of the magnetic detection probe 4. Prior to detection, the magnetic detection mode is selected using the mode switch 2. The output of the system, for example an indication of the proximity to the marker, position of the marker or distance from the marker to the probe is displayed on the screen 6 and may also be transmitted in the form of an audible signal 7.

The magnetic detection probe 4 may also be used to detect a lymph node 10 that contains magnetic tracer, for example a suspension of iron oxide nanoparticles used for lymph node detection. Magnetic tracers used for lymph node detection are generally colloidal suspensions of nanosized particles and typically comprise a magnetic core for example iron oxide and a biocompatible coating, for example dextran, carboxydextran, poly-ethylene glycol (PEG) or other polymer. Preferably the particle cores have a diameter of between 4 and 20 nanometres as measured using transmission electron microscopy (TEM). Preferably the coated particles have a hydrodynamic diameter of between 10 and 120 nanometres, and more preferably between 20 and 60 nanometres. Here, hydrodynamic diameter is defined as the diameter of a perfect solid sphere that would exhibit the same hydrodynamic friction as the particle of interest. Hydrodynamic diameter is commonly measured using dynamic light scattering.

However, for some patients it may not be possible or desirable to use the magnetic tracer, for example if they are contraindicated for iron oxide. In these cases, within the same procedure if required, the gamma probe 3 can be used to detect a lymph node 9 that contains radioactive tracer (for example a technetium 99 colloid of the kind used for sentinel lymph node detection). Prior to detection, the gamma detection mode is selected for example using the mode switch 2.

Rather than having to use a completely separate gamma detection system, the same base unit 1 can be used with both a magnetic probe 4 and a gamma probe 3, making the procedure more convenient for the surgeon and reducing the amount of equipment in the operating room.

Advantageously, the display uses similar user interface graphics and numerical or measurement output in order to communicate to the user, making it easier for the user to interpret the output of the probes than if they were using two completely different systems with different user interfaces. The user also needs to have a clear indication of which mode they are currently using. The interface preferably provides a common theme for the graphics and numerical output, but with a clear distinction between modes to aid the user in distinguishing the modes. In one embodiment, the graphical interface could be similar, but make use of a different colour or a different font or different probe graphic to distinguish modes. In another embodiment the same graphical user interface is used except that a symbol is displayed to indicate which mode is being used. In a further embodiment, the shape or colour or graphical quality of the border around the screen is changed to indicate the mode being used. A further aspect of the theme may be that the marker detection mode indicates a specific distance to the marker with a measuring unit such as millimetres or inches, while the tracer detection mode gives only a number (in arbitrary units) or graphical signal indicating the proximity or amount of tracer close to the probe. This is because the actual quantity of tracer accumulating in a region of interest such as a lymph node cannot be predicted and hence the signal response will vary with quantity as well as distance. In contrast, the signal from a marker will vary only with distance or direction as the amount of signal should not vary over time.

The audio output can also be made similar between modes so as to make the interpretation of the output easier for the user. The audio output may also contain elements of the sound that help the user to distinguish between the different modes. For example, while the same tone changes may be used to indicate signal strength, proximity or distance, a perturbation or modulation of the audio signal may be used to indicate which mode is being used. In one embodiment, a full tone may be used for one mode while a modulated or perturbed tone is used for another mode. In another embodiment, a dual tone is used to distinguish one mode from another. In another embodiment the shape of the waveform is modified to change the quality of the tone to distinguish one mode from another.

Figure 2 shows a further embodiment of the detection system according to the invention that can be used for detecting markers and tracers used in the body to mark lesions and lymph nodes.

The detection system comprises a base unit 1 and a gamma detection probe 3 that can communicate wirelessly with the base unit 1. The base unit 1 further comprises a visual display 6 for providing feedback and information to the user, and a mode switch 2 for switching between detection modes. The system further comprises one or more of a radar-based detection probe 14 connected to a base unit 1 via a cable or wired connection 20, and an RFID detection probe 16 connected to the base unit 1 via a cable or wired connection 20.

In some applications it may be advantageous to use a particular technology or system for marking and detecting lesions and a different system for tracing and detecting sentinel lymph nodes.

For example, the system of figure 2 shows a radar-based reflector implant 15 which is used to mark a lesion in the body. A radar based detection probe 14 is able to detect the implanted reflector implant 15 using a combination of RF electromagnetic waves and infra-red radiation. The probe 14 connects to the base unit 1 via a cable or wired connection 20.

The system of figure 2 further shows an RFID tag implant 18 which is used to mark a lesion in the body. An RFID detection probe 16 is able to detect the implanted RFID tag 18 using a detection coil 17. The probe 16 connects to the base unit 1 via a cable or wired connection 20.

Typically a system would comprise either a radar-based probe or an RFID probe as it is unlikely that two technologies would be used to localise the same lesion. However, it might be advantageous to have the option of other technology available for patients who have a contraindication to one technology.

The output from either probe is displayed on the screen 6 and may also be communicated audibly. The output may for example give an indication of the proximity to the marker, position of the marker or distance from the marker to the probe.

In the same procedure, when the surgeon wants to carry out sentinel lymph node tracer detection, the mode may be switched for example using the mode switch 2 to the gamma detection mode.

Within the scope of the invention it will be clear that any combination of a marker detection technology and any non-imaging tracer detection technology could be integrated into the system, but preferably either a magnetic or a radioactive tracer detection technology is used.

Table 1 below describes the characteristics of marker detection technologies that may be used as part of the system of the present invention, including the type of marker, the drive signal, and the signal that is sensed in order to detect the marker. One or more of these marker detection technologies can be combined within the system with either a magnetic or a radioactive or other non-imaging tracer detection technology.

**Table 1**

| **Marker detection technology and prior art reference** | **Marker description** | **Drive signal** | **Sense (detection) signal** |
|---|---|---|---|
| DC Magnetic field sensing US2019/019204 | Permanent magnet | None | DC magnetic field from the marker |
| DC Magnetic field sensing US2017/0319101 | Magnetised metal | None | DC magnetic field from the marker |
| DC magnetic excitation and detection | Ferromagnetic metal | DC field generated by permanent magnet or electromagnet | DC magnetic sensor e.g. Hall effect. Transient DC magnetic field from the marker |
| Sekino et al, 2018, Handheld magnetic probe with permanent magnet and Hall sensor for identifying sentinel lymp nodes in breast cancer patients | | | |
| AC Magnetic susceptometry | Ferromagnetic material | Alternating magnetic field | AC magnetic susceptometry. Change in alternating field induced by the presence of the marker |
| WO 2014/140567 | | | |
| EP2923216 | | | |
| Eddy current sensing- AC | Conductive material | Alternating magnetic field | Change in alternating field induced by the presence of Eddy currents induced in the marker |
| US2008/0097199 | | | |
| Resonant magnetic tag sensing | Resonant circuit such as capacitor and inductor | Alternating magnetic field | Change in alternating field induced by the presence of the marker |
| US10245119 | | | |
| RFID tag sensing | RFID tag - coil, ferrite core, electronic circuit | Short range RF energises tag | RF communication with transiently energised tag transceiver |
| US8973584 | | | |
| Radar (with infra-red) sensing | Electromagnetic reflector / antenna. Can be combined with infra-red activated switch to modify the antenna length | Wide band radar transmission. Can be accompanied by infra-red LED transmission | Wide band radar transmit/receive. Can be modulated by the signal from the IR LED |
| US2019/0365279 | | | |

More than two detection probes can be provided so as to provide the best set of detection tools possible for the surgeon. For example, it may be desirable to have different sizes or types of probe with the same technology for different types of procedure such as a thinner probe for more detailed work and a larger scanning probe for wide area detection.

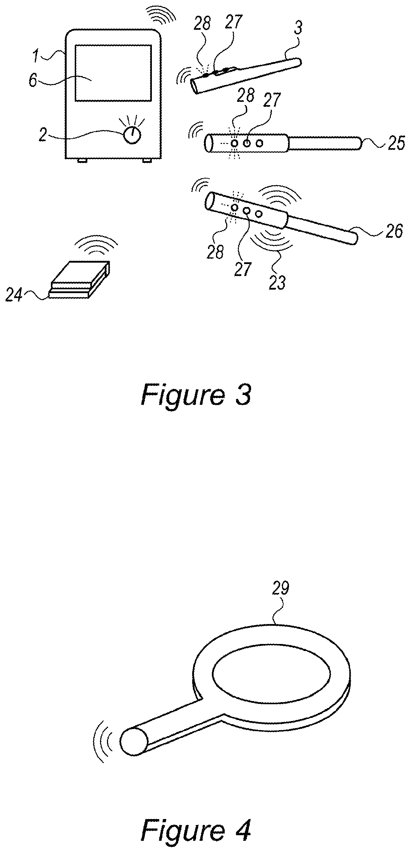

Figure 3 of the accompanying drawings shows a further detection system according to an embodiment of the present invention comprising a wireless magnetic detection probe of one diameter 25, a further wireless magnetic detection probe of a smaller diameter 26 and a wireless gamma probe 3.

Each of the probes can communicate with the base unit 1 wirelessly. Depending on the application or procedure the appropriate probe can be used for each part of the procedure without having to use a separate base unit.

The system in Figure 3 further comprises a footswitch 24 that communicates wirelessly with the base unit 1.

Preferably the wireless connection uses an established communication protocol such as Bluetooth, but a proprietary protocol may also be suitable.

The probes in figure 3 further comprise one or more switches 27 and visual indicators 28. The switches 27 may be used to control one or more functions of the system, for example to activate the probe, reset the display, change the display mode, mute the audio signal or another system function. The visual indicators 28 may be used to indicate the signal from the probe, the proximity or direction of the marker from the probe, or indicate battery life or another aspect of the function. The indicators preferably comprise LEDs.

Figure 3 also shows a probe 26 that provides haptic feedback 23 to the user in the form of a vibration, click , force or pressure to the user's hand. The haptic feedback may be used to indicate the signal from the probe, the proximity or direction of the marker from the probe, or another aspect of the system function.

In a further embodiment, gestures are used to control aspects of the system for example switching between modes or controlling the audio volume or display mode. For example, twisting, rotating, flicking, tapping in the air or pointing the probe may be used to provide a control signal to the base unit.

Figure 4 shows a scanning probe or wand able to detect the presence of a marker over a wider area of the body, for example with a scanning diameter of greater than 150mm and preferably greater than 250mm. This scanning probe is used to rapidly scan the patient's body prior to making an incision to locate the regions in which a marker is present prior to more localised, accurate detection with a handheld detection probe. This kind of scanning probe could be used for locating sentinel lymph nodes in malignant melanoma where the tracer if injected for example on the patient's back can migrate to a number of locations such as the axilla or the groin on either side of the patient. The scanning wand would enable the surgeon to locate the areas of the patient where an incision needs to be made to excise sentinel nodes. A handheld detection probe would then be used to precisely locate each node for excision. The scanning wand may be connected via a cable or wire or via a wireless connection to the base unit.

Referring to Figure 5 of the accompanying drawings, a block diagram of a detection system according to an embodiment of the invention is illustrated, the system comprising a magnetic tracer detection probe and a marker detection system using a different technology.

The system comprises a base unit, a magnetic tracer detection probe (C) and a marker detection probe (A) using another technology. The marker detection technology may be gamma detection also known as radioisotope detection; magnetic field detection; Eddy current detection; RF, radar or other electromagnetic detection; RFID tag detection; resonant magnetic tag detection, or another suitable non-imaging detection technology.

As illustrated by the boxes in Figure 5, the base unit comprises the caseworks or housing of the base unit, a main processing unit, a display, a loudspeaker or other audio output, user controls, analogue signal processing, and marker detection technology-specific analogue signal processing.

The user controls will typically comprise a mode select switch to select which probe and detection technology to use, a volume control to alter the volume of the audio output and a reset button to allow a detection mode to be reset or set to zero where required. The volume control may also comprise a feature to mute or unmute the audio output. The user controls may further comprise a footswitch with switches or other foot-operated controls to fulfil one or more of the user control functions.

The main processing unit comprises a microcontroller such as the STM32F769 microcontroller from STM Electronics. The microcontroller controls and interacts with a computer memory for example formed of SD RAM, and is part of a digital signal processing unit that may additionally comprise a Field Programmable Gate Array (FPGA). The microcontroller and FPGA generate an initial drive signal appropriate for the mode that has been selected via the mode select switch.

The base unit, user controls and main processing unit are common for both the tracer detection and the marker detection, thus reducing the amount of circuitry and software in the system and reducing the complexity and cost of the overall system.

This non-imaging magnetic tracer detection system is not to be confused with magnetic resonance imaging systems (MRI). MRI systems provide images based on the excitation of water molecules in tissue and can be used to image cancers for example breast cancers. Iron oxide particles are known in the art to provide enhanced contrast in MRI images following intravenous injection of the particles. In the present invention, however, the tracer is injected locally near a lesion, not intravenously, and the tracer is detected by non-imaging magnetic detection.

### Magnetic tracer detection mode

When the magnetic tracer detection mode has been selected, the initial drive signal is converted to an analogue signal via a digital to analogue converter (DAC) and then the drive circuit amplifies the signal and where necessary, filters the signal to optimise its purity. The amplified drive signal drives the magnetic field generator, to create the desired alternating field.

Also in the handheld tracer detection probe, there is a magnetic tracer signal detector, typically a coil or coils configured to detect magnetic fields in the vicinity of the probe, and preferably also comprising an amplifier to increase the magnitude of the signal before it is transmitted to the base unit. In the base unit, the tracer signal may be further amplified and filtered in the analogue signal processing unit before being digitised by the analogue to digital converter (ADC). Further signal processing may also happen in the digital signal processing unit, and this may involve any of filtering, demodulation, a lock-in amplifier and other means of isolating the signal from sources of noise and converting it into an output signal. The microcontroller communicates the output signal to the display and audio output (loudspeaker) to give the user an indication of the proximity of the probe to the tracer, position of the tracer or distance from the tracer to the probe.

### Marker detection mode

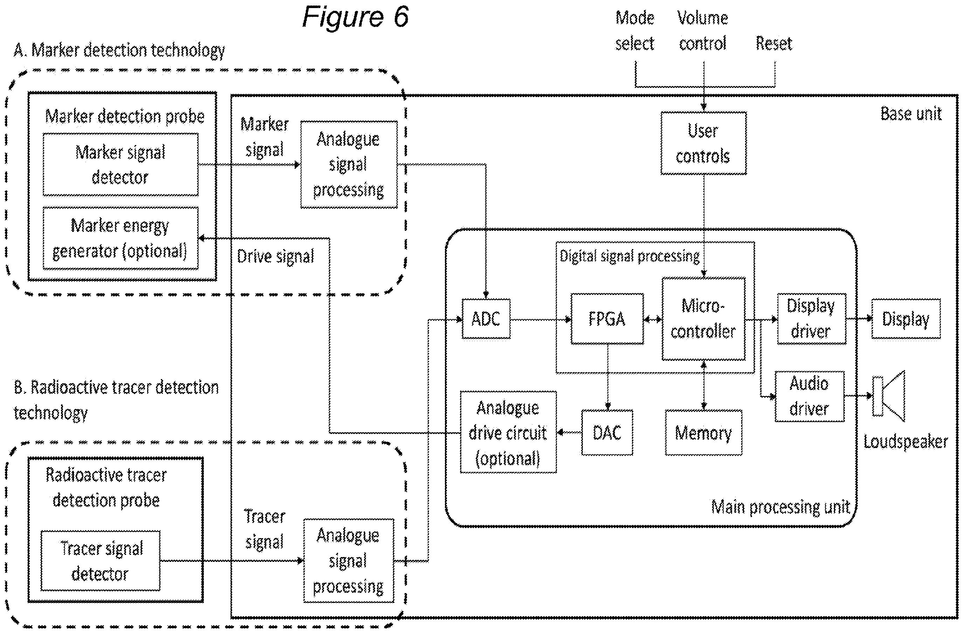

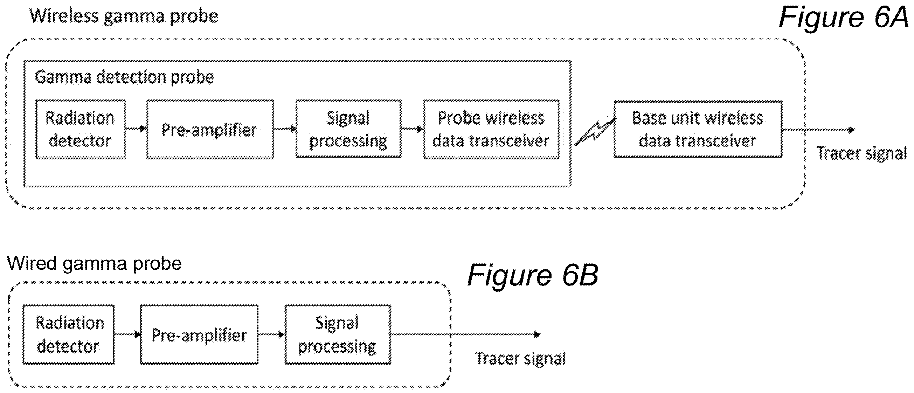

When the marker detection mode has been selected, where a drive is required, the initial drive signal is converted to an analogue signal via a digital to analogue converter (DAC). The drive signal is communicated to an analogue drive circuit specific to the marker detection technology employed where the signal may be amplified or filtered or be otherwise processed as required. The processed signal then drives an energy generator that is used to energise the marker. The marker detection technologies and their respective energy generators are described more specifically in the relation to Figures 6 to 12. Typically, though not always, the energy generator is in the handheld marker detection probe. However, in some embodiments the signal may be generated away from the probe, for example in a pad or mat underneath the patient. Not every marker detection technology utilises a drive signal and so it is to be appreciated that this part of the system is only employed where required.

Additionally, the handheld marker detection probe includes a marker signal detector, which detects the signal from the marker. The signal then undergoes analogue signal processing which may happen in the probe (not illustrated) or the base unit or in both the probe and the base unit. The processing may include filtering, amplification, demodulation, a lock-in amplifier and other techniques to isolate the desired signal form sources of unwanted noise. The signal detection and processing is described more specifically in the description relating to Figures 6 to 12. In the base unit, the marker signal may be processed by the analogue signal processing unit which is specific to the marker detection technology, before being digitised by the analogue to digital converter (ADC). The microcontroller communicates the output signal to the display and audio output (loudspeaker) via the display driver and audio driver respectively to give the user an indication of the proximity of the probe to the marker, position of the marker or distance from the marker to the probe.

In this exemplary arrangement, large parts of the base unit hardware are not specific to one or other detection technology but are used in the processing and communication of the signals from both different detection technology probes. These may include the user controls, the main processing unit, the display and display driver and the loudspeaker and audio driver. To have as much of the hardware in common as possible, some detection technology-specific elements are implemented in software rather than hardware. For example, each mode has technology-specific software code which runs when that mode is selected. In another example, specific areas of the FPGA may be programmed for each mode and used only when that mode is selected.

Figure 5A of the accompanying drawings illustrates a block diagram of a magnetic tracer detection technology (or probe) using one or more drive and sense coils.

The amplified drive signal coming from the microcontroller via the FPGA and DAC (see Figure 5) drives the magnetic field generator, which is a coil or coils configured to create an alternating magnetic field at the distal end of the handheld tracer detection probe, with the desired properties of frequency, amplitude and direction in order to excite the magnetic tracer. The magnetic tracer comprises superparamagnetic nanoparticles typically comprising iron oxide cores.

Also in the handheld tracer detection probe, there is a magnetic tracer signal detector, comprising a sense coil or coils configured to detect magnetic fields coming from the magnetic tracer in the vicinity of the probe, or to detect distortions in the magnetic field around the probe caused by the particles. Preferably the probe also comprises filtering to improve the quality of the signal and reduce the unwanted noise, and a first stage amplifier to increase the magnitude of the tracer signal before it is transmitted to the base unit.

Figure 6 illustrates a block diagram of the detection system according to an embodiment of the invention which comprises a radioactive tracer detection probe and a marker detection system using a different technology.

The system comprises a base unit, a radioactive tracer detection probe and a marker detection probe using another technology. The marker detection technology may be gamma detection also known as radioisotope detection; magnetic field detection; Eddy current detection; RF, radar or other electromagnetic detection; RFID tag detection; resonant magnetic tag detection, or another suitable marker detection technology.

The base unit, user controls and main processing unit are as described for Figure 5.

### Radioactive tracer detection mode

In this embodiment, when the radioactive tracer detection mode has been selected, no drive signal is required as the radiation sensor is a passive sensor.

In the handheld tracer detection probe, there is a radioactive tracer signal detector, and a signal processing unit, both described in more detail in Figures 6A and 6B. In the base unit, the tracer signal may be processed by the analogue signal processing unit which is specific to the marker detection technology, before being digitised by the analogue to digital converter (ADC). Further signal processing may also happen in the digital signal processing unit, and this may involve any of filtering, demodulation, a lock-in amplifier and other means of isolating the signal from sources of noise and converting it into an output signal. The microcontroller communicates the output signal to the display and audio output (loudspeaker) to give the user an indication of the proximity of the probe to the tracer, position of the tracer or distance from the tracer to the probe.

Figures 6A and 6B illustrate block diagrams of a tracer detection system comprising a radioactive tracer detection technology, also known as gamma detection, with a wireless probe (Figure 6A) and a wired probe (Figure 6B).

The radioactive tracer detection probe typically comprises a radiation detector to generate a small electrical signal in response to incident radiation, a pre-amplifier to increase the magnitude of the signal, and a signal processing unit to filter or otherwise isolate the signal from unwanted noise. The signal is then transmitted to the base unit, either via a cable ('wired' probe) or via a wireless link. The tracer signal then connects to the main processing unit via the ADC and the signal is processed as described in the descriptions of Figures 5 and 6.

The radiation detector comprises a crystal made from cadmium zinc telluride or any other semiconductor material suitable for detecting photon radiation, or a scintillation counter to detect radiation emitted in the vicinity of the probe.

Typically the radioactive tracer used with the probe is a colloid or injectable solution labelled with a Technetium 99m isotope. The radiolabelled tracer particles are sized for take-up from tissue by the lymphatic system and subsequent filtering out in draining lymph nodes. Typically they have a hydrodynamic particle diameter of between 5 and 100 nanometres.

### Marker detection mode

The marker detection mode of the system of Figure 6 operates in a similar way to that described in relation to Figure 5.

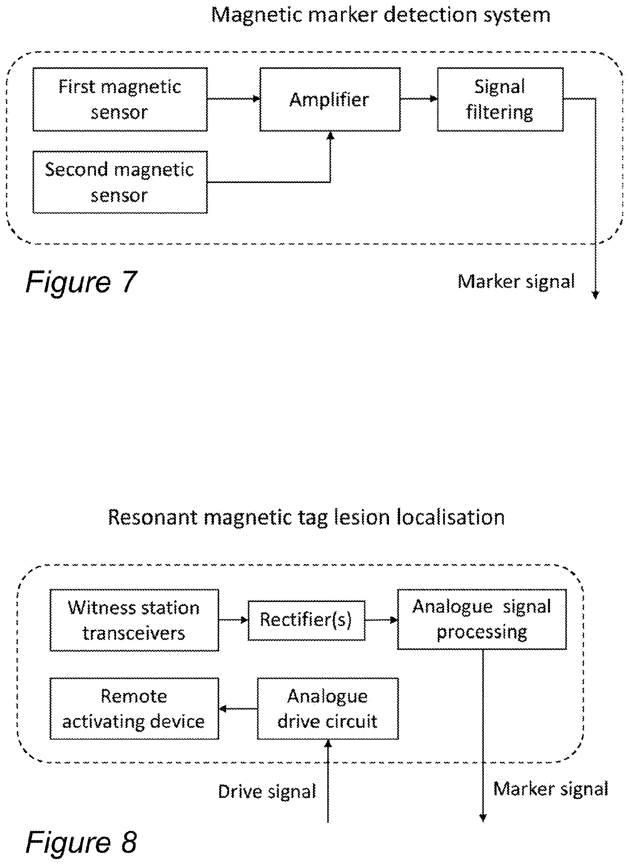

Figure 7 illustrates a block diagram of a marker detection system comprising a magnetic detection technology. This detection technology does not require a drive signal and detects permanent magnet markers, magnetic markers that generate a DC magnetic field, or markers that make detectable changes in a DC magnetic field.

Typically, the magnetic marker will comprise a small permanent magnet that is coated to achieve biocompatibility. The marker may alternatively comprise a ferromagnetic material that has been magnetised so that it exhibits some residual or remanent magnetism and generates its own magnetic field.

The detection probe typically comprises two magnetic sensors. The sensors may be of any suitable type for detecting a DC or slowly alternating magnetic field. Suitable sensors include but are not limited to Hall effect, search coil sensor, fluxgate magnetometer, magnetoresistance (e.g. AMR, GMR), magnetoimpedance, magnetotransistor, and magneto-optical sensors.

One sensor is located close to the distal end of the probe for sensing, and the other is located away from the distal end of the probe to detect the background field or to provide a second field measurement for comparison with the measurement from the first sensor. The magnetic signals are amplified, filtered and processed (not shown) to isolate the signal from unwanted noise. The marker signal is then fed into the main processing unit via the ADC.

The marker detection system of Figure 7 may additionally comprise a permanent magnet (not shown) near the distal end of the probe which magnetises the implantable marker. In this case the marker is preferably formed from a ferromagnetic material rather than being a permanent magnet marker. Advantageously, the first magnetic sensor is placed at a null point in the field generated by the permanent magnet so that it is more sensitive when detecting the marker. This type of detection system is thus called a DC magnetic excitation and detection system because both the exciting and detected field are DC field.

Note that these systems are not to be confused with magnetic resonance imaging systems (MRI). MRI systems provide images based on the excitation of water molecules in tissue and can be used to image cancers for example breast cancers. Metallic and magnetic materials can be seen on MRI images. Many metallic markers are known in the art for example biopsy markers which often comprise non-ferrous metals that can be seen under MRI and can be known as `MRI detectable' markers. However, these markers are usually not suitable for non-imaging magnetic detection because they contain minimal ferromagnetic material. Furthermore, detectability under MRI does not imply detectability by non-imaging magnetic detection. Ferromagnetic materials may generate a flare or void artefact on MRI imaging, for which reason, these materials are not generally imaged using MRI, however, for non-imaging magnetic detection, ferromagnetic materials are desirable, for example, those with a high relative permeability. In the present invention, the marker is detected by non-imaging magnetic detection which is unrelated to MRI imaging and uses different physical principles. For example, while MRI images are based on the excitation of water molecules in tissue, the markers of the present invention either generate their own magnetic field or generate a magnetic field through being excited by an alternating field generated by the marker detection system, and the magnetic field is detected using a non-imaging detection technology such as magnetic susceptometry, or other DC or AC magnetic field detection techniques.

Figure 8 illustrates a block diagram of another marker detection system comprising a resonant magnetic tag detection technology. In this system, the drive signal drives a remote activating device typically comprising a coil driven by an alternating signal. The coil may be conveniently positioned in a mat underneath the patient such that the field extends to the area in the patient where the implantable tag is. One or more resonant tags are excited by the alternating magnetic field and generate sidebands at defined frequencies. The tags typically comprise a coil and suitable electronic components to create a resonance in the presence of an alternating magnetic field, for example a capacitor, inductor or combination of the two. The marker may also comprise a non-linear component such as a Schottky diode or other diode such that a non-linear harmonic response is generated. A number of witness stations detect the sidebands or harmonic signals and the combination of these signals can be processed to provide information such as the vector position of the tag. The processing steps may include being rectified prior to further analogue signal processing. This information is transmitted to the base unit.

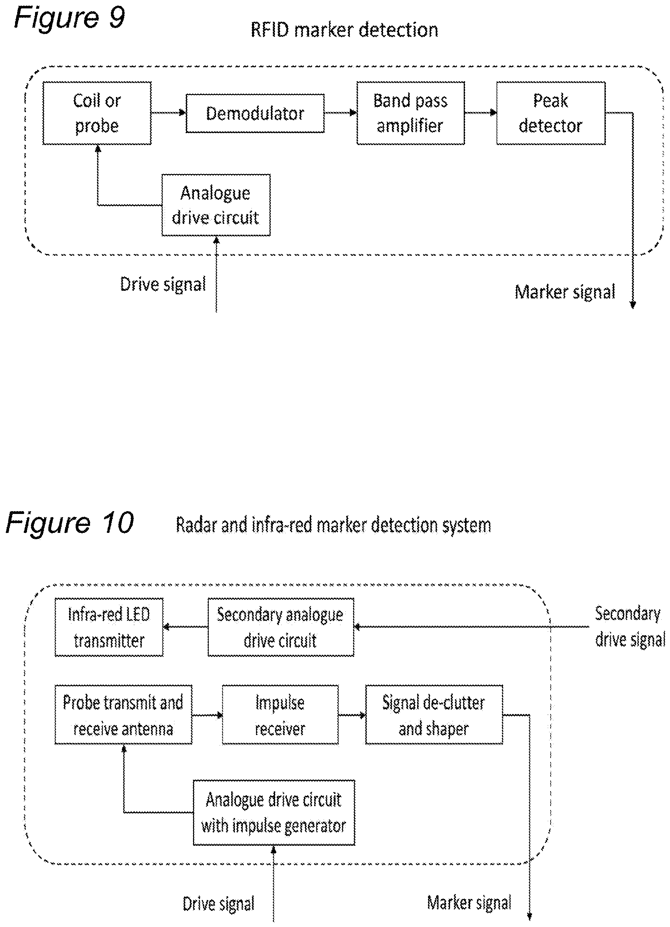

Figure 9 of the accompanying drawings illustrates a block diagram of an alternative marker detection system comprising an RFID detection technology. In one example of this RFID system, the analogue drive circuit excites a coil to generate an RF signal which is able to excite a resonance in the passive integrated transponder (PIT) tag, also commonly known as an RFID tag. The signal from the PIT tag is demodulated and filtered before being transmitted to the base unit. Typically, the marker is an RFID tag of the sort implanted in pets to identify them, and comprises a coil, with or without a ferrite core, and a microchip (not shown). When energised by an RF signal, the microchip transmits information to enable the proximity or position or distance between the tag and a detection probe to be determined.

Figure 10 illustrates a block diagram of another type of marker detection system comprising a radar detection technology also known as an RF electromagnetic wave detection technology. The drive signal drives an impulse generator which transmits radar impulses via an antenna on a handheld probe. These radar impulses are reflected by the marker which comprises a radar reflector. The probe antenna also receives these impulses at the impulse receiver and the signal is de-cluttered and shaped to isolate the desired marker signal form unwanted noise. The signal is then transmitted to the base unit.

Furthermore, the probe may comprise a secondary drive signal originating from the main control unit (connection not shown) which via an analogue drive circuit drives an infra-red LED that transmits a different, preferably lower frequency alternating infra-red signal that illuminates the tag. The tag may further comprise a photodiode or phototransistor which when illuminated modifies the effective size of the reflector, for example by electrically joining two reflector sections, such that the reflected radar signal is altered, for example in intensity. The net effect is that the received radar signal is modulated or modified by the infra-red signal, and this modulated signal is only seen when the tag is illuminated by the infra-red LED, providing more specificity to the detection of the tag.

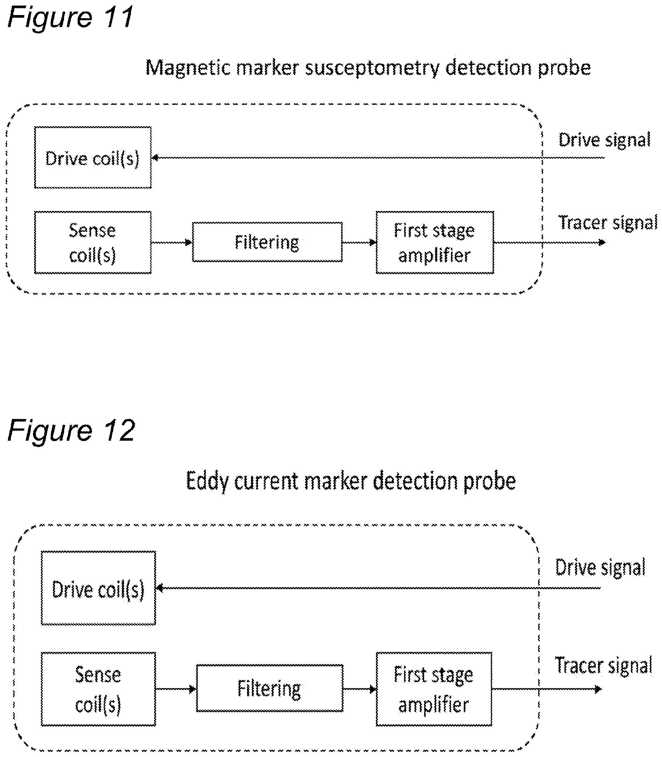

Figure 11 illustrates a block diagram of yet another example of a marker detection system comprising magnetic susceptometry detection technology using one or more drive and sense coils to detect a magnetically responsive marker. This technology is similar to the technology illustrated in Fig 5A for detection of magnetic tracers, but the probe design and signal processing may be optimised differently for detection of a magnetic marker.

The amplified drive signal coming from the microcontroller via the FPGA and DAC drives the magnetic field generator, which is a coil or coils configured to create an alternating magnetic field at the distal end of the handheld marker detection probe, with the desired properties of frequency, amplitude and direction in order to excite the magnetic marker.

Also in the handheld tracer detection probe, there is a magnetic marker signal detector, comprising a sense coil or coils configured to detect magnetic fields coming from the magnetic marker in the vicinity of the probe, or to detect distortions in the magnetic field around the probe caused by the marker. Preferably the probe also comprises filtering to improve the quality of the signal and reduce the unwanted noise, and a first stage amplifier to increase the magnitude of the marker signal before it is transmitted to the base unit.

Suitable materials for the magnetic marker include ferromagnetic materials such as iron, nickel or cobalt, or alloys or combinations of one or more of these materials; magnetic amorphous alloys; bulk metallic glasses; materials exhibiting a large Barkhausen jump in their magnetisation curve; and superparamagnetic particles. Typically these materials will have a high relative permeability (denoted commonly as µᵣ) of greater than 100, and preferably greater than 500. The marker preferably has a high magnetic mass susceptibility, χ_{ρ}. The χ_{ρ} should be greater than or equal to 0.05 m³kg⁻¹, preferably greater than or equal to 0.1 m³kg⁻¹ and more preferably greater than or equal to 1 m³kg⁻¹.

Figure 12 illustrates a block diagram of another marker detection system comprising an Eddy current detection technology using one or more drive and sense coils to detect a magnetically responsive marker. An alternating magnetic field generated by one or more coils in the probe induces Eddy currents in a conductive marker.

The amplified Drive signal coming from the microcontroller via the FPGA and DAC drives the magnetic field generator, which is a coil or coils configured to create an alternating magnetic field at the distal end of the handheld tracer detection probe, with the desired properties of frequency, amplitude and direction in order to excite the magnetic marker. The field induces Eddy currents in the conductive marker.

Also in the handheld tracer detection probe, there is an Eddy current marker signal detector, comprising a sense coil or coils configured to detect induced magnetic fields coming from the Eddy current marker in the vicinity of the probe, or to detect distortions in the magnetic field around the probe caused by the Eddy current marker. Preferably the probe also comprises filtering to improve the quality of the signal and reduce the unwanted noise, and a first stage amplifier to increase the magnitude of the marker signal before it is transmitted to the base unit. Typically the Eddy current marker is constructed at least in part from a material with a high conductivity such as gold, silver or copper, although a biocompatible material such a gold is preferable for the outer surface.

Within the scope of the invention, any tracer detection probe and technology can be combined with any other tracer detection probe and technology. However, certain combinations are preferred as described in the Table 2 below:

**Table 2**

| **Tracer detection probe technology** | **Marker detection probe technology** | **Advantage of this combination** |
|---|---|---|
| Gamma radioisotope detection | Magnetic susceptometry - AC (system may also include magnetic tracer detection) | Allows magnetic tracer detection when radioisotope tracer is contra-indicated and vice versa. |
| Gamma radioisotope detection | Radar (with infra-red) sensing | Adds tracer detection capability for sentinel node detection which cannot be achieved with radar with infra-red detection, and cannot be integrated readily into a radar probe |
| Gamma radioisotope detection | RFID tag sensing | Adds tracer detection capability for sentinel node detection which cannot be achieved with RFID detection and cannot be integrated readily into an RFID probe |
| Gamma radioisotope detection | Resonant magnetic tag sensing | Adds tracer detection capability for sentinel node detection which cannot be achieved with resonant magnetic tag sensing |
| Gamma radioisotope detection | Magnetic field sensing - DC | Adds tracer detection capability for sentinel node detection which cannot be achieved with DC magnetic field sensing |
| Gamma radioisotope detection | Eddy current sensing- AC | Adds tracer detection capability for sentinel node detection which cannot be achieved with AC Eddy current sensing and cannot be integrated readily into an Eddy current sensing probe |
| Magnetic susceptometry - AC | Magnetic field sensing - DC | Adds tracer detection capability for sentinel node detection which cannot be achieved with DC magnetic field sensing |

## Claims

1. A detection system for locating markers or tracers in the body, the system comprising:
a base unit (1) and at least two detection probes (3, 4), wherein a first detection probe (4) uses a first non-imaging detection technology to detect an implantable marker (8) and the detection system is **characterised in that** a second detection probe (3) uses a second different, non-imaging detection technology to detect an injectable tracer (9);
and the base unit (1) is capable of receiving detection signals and providing detection information to a user from both probes (3, 4).

2. The detection system of claim 1 wherein the implantable marker (8) comprises detection technology to detect and locate markers marking the site of a lesion of interest ("lesion localisation") and the injectable tracer is for lymph node (10) detection.

3. The detection system of claim 1 or claim 2 wherein the base unit (1) has components common to both detection technologies and components specific to each different detection technology.

4. The detection system of claim 3 wherein the components common to both detection technologies are selected from the group consisting of a main processing unit; a signal processing unit; a display (6); an audio output (7); an analogue to digital convertor (ADC); a digital to analogue convertor (DAC); data storage memory; user controls and caseworks (11) or any combinations thereof.

5. The detection system of any one of the preceding claims, wherein the base unit (1) is provided with a drive signal source for providing a drive signal to one or both probes (3,4) to excite an energy source in the probe for exciting the marker or tracer.

6. The detection system of any one of the preceding claims wherein the marker (8) or tracer (9) emits a signal which is detected by a sensor in the first (4) or second (3) probe which is transmitted to and received by the base unit (1).

7. The detection system of any one of the preceding claims wherein at least one of the probes (3, 4) communicates with the base station wirelessly.

8. The detection system of any one of the preceding claims wherein the first probe (4) is a magnetic probe, or an electromagnetic probe, or a RFID probe, or a radar-based probe and the second probe (3) is a gamma probe.

9. The detection system of claim 8 wherein the first probe (4) is a wired magnetic probe, or a wired radar-based probe, or a wired RFID probe, or a wireless magnetic probe and the second probe (3) is a wireless gamma probe.

10. The detection system of any one of claims 1-7, wherein the non-imaging marker detection technology used in the first probe (4) is selected from: gamma detection; magnetic detection; electromagnetic detection; RFID detection; DC magnetic field sensing; AC magnetic susceptometry; DC magnetic excitation and detection; resonant magnetic tag detection; Radar detection; and infra-red detection, or any combinations thereof.

11. The detection system of any one of claims 1-7 wherein the non-imaging tracer detection technology used in the second probe (3) is selected from: gamma detection; DC magnetic excitation and detection; DC magnetic field sensing; and AC magnetic susceptometry.

12. The detection system of any one of the preceding claims wherein the system includes a mode select switch (2) to switch between a marker detection probe mode and a tracer detection probe mode.

13. The detection system of claim 10 wherein the mode select switch (2) is selected from a switch or dial on the base unit (1), a footswitch (40), a control on the probe or be via voice or gesture control.

14. The detection system of claim 10 or claim 11 wherein the base unit (1) includes a visual or audio display (6, 7) providing a different theme dependent upon the selected mode to aid differentiation between the modes of detection.

15. The detection system of any one of the preceding claims wherein the base unit (1) includes an amplifier to amplify the detection signals received from the detection probes (3,4).

16. The detection system of any one of the preceding claims wherein the base unit (1) receives signals from one or both probes (3, 4) to provide an output indicating at least one of the position of the marker (8) or tracer (9) and the distance of the marker or tracer from the probe or the proximity of the marker or tracer to the probe.

17. The detection system of any one of the preceding claims wherein the base unit (1) comprises a housing (11), a processing unit disposed in the housing, a display (6), an audio interface (7) and one or more controls (2, 30, 32) in electronic communication with the processing unit, wherein one or more of the detection probes (3, 4) is in electronic communication with the processing unit.

## Patentansprüche

1. Erfassungssystem zum Lokalisieren von Markern oder Tracern in dem Körper, das System weist auf:
eine Basiseinheit (1) und mindestens zwei Erfassungsmessfühler (3, 4), bei dem ein erster Erfassungsmessfühler (4) eine erste nicht-bildgebende Erfassungstechnologie zum Erfassen eines implantierbaren Markers (8) verwendet und das Erfassungssystem **dadurch gekennzeichnet ist, dass** ein zweiter Erfassungsmessfühler (3) eine zweite, nicht-bildgebende Erfassungstechnologie zum Erfassen eines injizierbaren Tracers (9) verwendet;
und die Basiseinheit (1) dazu fähig ist, Erfassungssignale zu empfangen und Erfassungsinformationen beider Messfühler (3, 4) für einen Benutzer bereitzustellen.

2. Erfassungssystem nach Anspruch 1, bei dem der fest einsetzbare Marker (8) Erfassungstechnologie zum Erfassen und Lokalisieren von Markern aufweist, die die Lage einer Läsion, die von Interesse ist, kenntlich macht ("Läsionslokalisierung") und der injizierbare Tracer zur Lymphknotenerfassung (10) dient.

3. Erfassungssystem nach Anspruch 1 oder 2, bei dem die Basiseinheit (1) Komponenten, die beide Erfassungstechnologien gemeinsam haben, und Komponenten, die für jede verschiedene Erfassungstechnologie spezifisch sind, aufweist.

4. Erfassungssystem nach Anspruch 3, bei dem die Komponenten, die beide Erfassungstechnologien gemeinsam haben, ausgewählt sind aus der Gruppe, bestehend aus: einer Hauptverarbeitungseinheit, einer Signalverarbeitungseinheit; einem Display (6); einer Tonausgabe (7); einem Analog-Digital-Wandler (ADC); einem Digital-Analog-Wandler (DAC); Datenspeicher; Benutzersteuerungen und Gehäusen (11) oder jegliche Kombination derselben.

5. Erfassungssystem nach einem der vorhergehenden Ansprüche, bei dem die Basiseinheit (1) mit einer Treibersignalquelle zum Liefern eines Treibersignals an einen oder beide Messfühler (3, 4) vorgesehen ist, zum Anregen einer Energiequelle in dem Messfühler zum Anregen des Markers oder Tracers.

6. Erfassungssystem nach einem der vorhergehenden Ansprüche, bei dem der Marker (8) oder der Tracer (9) ein Signal ausgeben, das durch einen Sensor in dem ersten (4) oder zweiten (3) Messfühler erfasst wird, das zu der Basiseinheit (1) übertragen wird und durch dieselbe empfangen wird.

7. Erfassungssystem nach einem der vorhergehenden Ansprüche, bei dem mindestens einer der Messfühler (3, 4) drahtlos mit der Basisstation kommuniziert.

8. Erfassungssystem nach einem der vorhergehenden Ansprüche, bei dem der erste Messfühler (4) ein magnetischer Messfühler, oder ein elektromagnetischer Messfühler, oder ein RFID Messfühler, oder ein radarbasierter Messfühler ist und der zweite Messfühler (3) ein Gamma-Messfühler ist.

9. Erfassungssystem nach Anspruch 8, bei dem der erste Messfühler (4) ein drahtgebundener magnetischer Messfühler, oder ein drahtgebundener radarbasierter Messfühler, oder ein drahtgebundener RFID Messfühler, oder ein drahtloser magnetischer Messfühler ist und der zweite Messfühler (3) ein drahtloser Gamma-Messfühler ist.

10. Erfassungssystem nach einem der Ansprüche 1-7, bei dem die nicht-bildgebende Markererfassungstechnologie, die in dem ersten Messfühler (4) verwendet wird, ausgewählt ist aus: Gamma-Erfassung; magnetischer Erfassung; RFID Erfassung; Gleichstrommagnetfeldabtasten; magnetischer Wechselstrom-Suszeptometrie; magnetischer Gleichstromerregung und -erfassung; magnetischer Resonanzmarkierungserfassung; Radarerfassung; und Infraroterfassung, oder jede Kombination derselben.

11. Erfassungssystem nach einem der Ansprüche 1-7, bei dem die nicht-bildgebende Tracererfassungstechnologie, die in dem zweiten Messfühler (3) verwendet wird, ausgewählt ist aus: Gamma-Erfassung; magnetischer Gleichstromerregung und -erfassung; Gleichstrommagnetfeldabtasten; und magnetischer Wechselstrom-Suszeptometrie.

12. Erfassungssystem nach einem der vorhergehenden Ansprüche, bei dem das System einen Modusauswahlschalter (2) zum Umschalten zwischen einem Markererfassungsmessfühlermodus und einem Tracererfassungsmessfühlermodus aufweist.

13. Erfassungssystem nach Anspruch 10, bei dem der Modusauswahlschalter (2) aus einem Schalter oder Wählsystem an der Basiseinheit (1), einem Fußschalter (40), einer Steuerung an dem Messfühler oder durch Sprachsteuerung oder Gestensteuerung ausgewählt ist.

14. Erfassungssystem nach Anspruch 10 oder 11, bei dem die Basiseinheit (1) ein visuelles oder Audiodisplay (6,7) aufweist, die ein unterschiedliches Motiv vorsieht, abhängig von dem gewählten Modus, zum Unterstützen der Unterscheidung zwischen den Erfassungsmodi.

15. Erfassungssystem nach einem der vorhergehenden Ansprüche, bei dem die Basiseinheit (1) einen Verstärker zum Verstärken der Erfassungssignale, die von den Erfassungsmessfühlern (3, 4) empfangen werden, aufweist.

16. Erfassungssystem nach einem der vorhergehenden Ansprüche, bei dem die Basiseinheit (1) Signale von einem oder beiden Messfühlern (3, 4) empfängt, zum Bereitstellen einer Ausgabe, die mindestens eines aus der Position des Markers (8) oder Tracers (9) und dem Abstand des Markers oder Tracers von dem Messfühler oder der Nähe des Markers oder des Tracers zu dem Messfühler angibt.

17. Erfassungssystem nach einem der vorhergehenden Ansprüche, bei dem die Basiseinheit (1) ein Gehäuse (11), eine Verarbeitungseinheit, die in dem Gehäuse angeordnet ist, ein Display (6), ein Audio-Interface (7) und ein oder mehrere Steuerungselemente (2, 30, 32) zur elektronischen Verbindung mit der Verarbeitungseinheit aufweist, bei dem einer oder mehrere der Erfassungsmessfühler (3, 4) in elektronischer Verbindung mit der Verarbeitungseinheit stehen.

## Revendications

1. Système de détection pour localiser des marqueurs ou des traceurs dans le corps, le système comprenant :
une unité de base (1) et au moins deux sondes de détection (3, 4), dans lequel une première sonde de détection (4) utilise une première technologie de détection sans imagerie pour détecter un marqueur implantable (8) et le système de détection est **caractérisé en ce qu'**une seconde sonde de détection (3) utilise une seconde technologie de détection sans imagerie différente pour détecter un traceur injectable (9) ;
et l'unité de base (1) est capable de recevoir des signaux de détection et de fournir des informations de détection à un utilisateur à partir des deux sondes (3, 4).

2. Système de détection selon la revendication 1, dans lequel le marqueur implantable (8) comprend une technologie de détection pour détecter et localiser des marqueurs marquant le site d'une lésion d'intérêt (« localisation de lésion ») et le traceur injectable est destiné à la détection de ganglions lymphatiques (10).

3. Système de détection selon la revendication 1 ou la revendication 2, dans lequel l'unité de base (1) a des composants communs aux deux technologies de détection et des composants spécifiques à chaque technologie de détection différente.

4. Système de détection selon la revendication 3, dans lequel les composants communs aux deux technologies de détection sont choisis dans le groupe constitué par : une unité de traitement principale ; une unité de traitement de signaux ; un écran d'affichage (6) ; une sortie audio (7) ; un convertisseur analogique-numérique (ADC) ; un convertisseur numérique-analogique (DAC) ; une mémoire de stockage de données ; des commandes utilisateur et des études de cas (11) ou toute combinaison de ceux-ci.

5. Système de détection selon l'une quelconque des revendications précédentes, dans lequel l'unité de base (1) est pourvue d'une source de signal d'attaque pour fournir un signal d'attaque à l'une ou aux deux sondes (3, 4) pour exciter une source d'énergie dans la sonde pour exciter le marqueur ou le traceur.

6. Système de détection selon l'une quelconque des revendications précédentes, dans lequel le marqueur (8) ou le traceur (9) émet un signal qui est détecté par un capteur dans la première (4) ou la seconde (3) sonde, qui est transmis à et reçu par l'unité de base (1).

7. Système de détection selon l'une quelconque des revendications précédentes, dans lequel au moins l'une des sondes (3, 4) communique sans fil avec la station de base.

8. Système de détection selon l'une quelconque des revendications précédentes, dans lequel la première sonde (4) est une sonde magnétique, ou une sonde électromagnétique, ou une sonde RFID, ou une sonde radar et la seconde sonde (3) est une sonde gamma.

9. Système de détection selon la revendication 8, dans lequel la première sonde (4) est une sonde magnétique câblée, ou une sonde radar câblée, ou une sonde RFID câblée, ou une sonde magnétique sans fil et la seconde sonde (3) est une sonde gamma sans fil.

10. Système de détection selon l'une quelconque des revendications 1 à 7, dans lequel la technologie de détection de marqueur sans formation d'image utilisée dans la première sonde (4) est choisie parmi : la détection gamma ; la détection magnétique ; la détection électromagnétique ; la détection RFID ; la détection de champ magnétique en courant continu ; la susceptométrie magnétique en courant alternatif ; l'excitation et la détection magnétique en courant continu ; la détection d'étiquette magnétique résonnante ; la détection radar ; et la détection infrarouge, ou toute combinaison de celles-ci.

11. Système de détection selon l'une quelconque des revendications 1 à 7, dans lequel la technologie de détection de traceur sans formation d'image utilisée dans la seconde sonde (3) est choisie parmi : la détection gamma ; l'excitation et la détection magnétique en courant continu ; la détection de champ magnétique en courant continu ; et la susceptométrie magnétique en courant alternatif.

12. Système de détection selon l'une quelconque des revendications précédentes, dans lequel le système comprend un commutateur de sélection de mode (2) pour commuter entre un mode de sonde de détection de marqueur et un mode de sonde de détection de traceur.

13. Système de détection selon la revendication 10, dans lequel le commutateur de sélection de mode (2) est sélectionné parmi un commutateur ou un cadran sur l'unité de base (1), un commutateur au pied (40), une commande sur la sonde ou par le biais d'une commande vocale ou gestuelle.

14. Système de détection selon la revendication 10 ou la revendication 11, dans lequel l'unité de base (1) comprend un affichage visuel ou sonore (6, 7) fournissant un thème différent en fonction du mode sélectionné pour faciliter la différenciation entre les modes de détection.

15. Système de détection selon l'une quelconque des revendications précédentes, dans lequel l'unité de base (1) comprend un amplificateur pour amplifier les signaux de détection reçus des sondes de détection (3, 4).

16. Système de détection selon l'une quelconque des revendications précédentes, dans lequel l'unité de base (1) reçoit des signaux de l'une ou des deux sondes (3, 4) pour fournir une sortie indiquant au moins l'une de la position du marqueur (8) ou du traceur (9) et de la distance du marqueur ou du traceur par rapport à la sonde ou de la proximité du marqueur ou du traceur par rapport à la sonde.

17. Système de détection selon l'une quelconque des revendications précédentes, dans lequel l'unité de base (1) comprend un boîtier (11), une unité de traitement disposée dans le boîtier, un écran d'affichage (6), une interface audio (7) et une ou plusieurs commandes (2, 30, 32) en communication électronique avec l'unité de traitement, dans lequel une ou plusieurs des sondes de détection (3, 4) sont en communication électronique avec l'unité de traitement
